# EUROPEAN PATENT APPLICATION

(11) **EP 4 487 779 A1**
(43) Date of publication of application: **08.01.2025**
(21) Application number: 23763605.5
(22) Date of filing: 09.01.2023
(51) Int. Cl.: A61B 5/346, A61B 5/00, G16H 50/20

(54) **DISEASE DIAGNOSIS METHOD**

(30) Priority: 03.03.2022 KR 20220027221
(71) Applicant: VUNO INC., Seoul 06541 (KR)
(72) Inventor: CHANG, Mineok, Seoul 06655 (KR); JOO, Sunghoon, Seoul 04778 (KR); LEE, Sungjae, Seoul 04582 (KR); KIM, Kyung Geun, Seoul 06288 (KR); NA, Yeongyeon, Seoul 08026 (KR)
(74) Representative: AWA Sweden AB
(86) International application number: PCT/KR2023/000378
(87) International publication number: WO 2023/167406

(57) **Abstract**

According to an embodiment of the present disclosure, disclosed is a method for evaluating qualities of electrocardiogram data and diagnosing a disease only with a signal having an excellent quality. Specifically, according to the present disclosure, a computing device evaluates qualities of electrocardiogram data obtained from a plurality of leads, excludes electrocardiogram data obtained from at least one lead among the plurality of leads based on the evaluation of the qualities of the electrocardiogram data, and performs electrocardiogram analysis based on remaining electrocardiogram data by using a pre-learned electrocardiogram analysis model.

## Description

### [Technical Field]

The present disclosure relates to a method for diagnosing a disease, and particularly, to a method which evaluates the quality of electrocardiogram data from a multi-channel electrocardiogram measurement result, and diagnoses a disease only with a signal having an excellent quality.

### [Background Art]

Although a research on a disease diagnosis method through biological signals is broadly conducted by using a deep learning-based algorithm, accuracy of methods already developed is decreased due to the characteristics of the biological signal, that is, noise generated when a measurement length is long and the number of input channels is large.

In particular, in the diagnosis of the disease through electrocardiogram, when using the Holter Recorder, Patch Recorder, and Patient Monitoring Device for electrocardiogram measurement, due to a feature that the electrocardiogram should be measured for one hour as a short period and a long time corresponding to several weeks as a long period with respect to a subject performing a physical activity, electrocardiogram data measured in that method are highly likely to contain a number of noise.

In the deep learning based electrocardiogram diagnosis algorithm in related art, since it is possible to diagnose the electrocardiogram only by inputting electrocardiogram data having leads of a number which matches the number of learned leads, there is a problem in that diagnosis becomes difficult when a signal having a bad quality, in which noise at a predetermined level or more is generated or noise is not measured is measured, which should not be used in any specific lead.

Therefore, in the art, there is a demand on a method for automatically selecting a normal signal in disease diagnosis through electrocardiogram and diagnosing a disease by using the normal signal.

Korean Patent Registration No. 10-1799194 (November 13, 2017) discloses an arrhythmia diagnosis apparatus using electrocardiogram signal.

### [Disclosure]

### [Technical Problem]

The present disclosure is contrived in response to the above-described background art, and an object of the present disclosure is to provide a method which evaluates the quality of an electrocardiogram signal constituted by multiple leads, analyzes electrocardiogram using only a signal having excellent quality, and diagnoses a disease.

### [Technical Solution]

In order to implement the above-described object, according to an embodiment of the present disclosure, disclosed is a method for evaluating qualities of electrocardiogram data, and performing electrocardiogram analysis. The method may include: evaluating qualities of electrocardiogram data obtained from a plurality of leads; excluding electrocardiogram data obtained from at least one lead among the plurality of leads based on the evaluation of the qualities of the electrocardiogram data; and performing electrocardiogram analysis based on remaining electrocardiogram data by using a pre-trained electrocardiogram analysis model.

In an alternative embodiment, the evaluating of the qualities of electrocardiogram data obtained from the plurality of leads may include dividing the electrocardiogram data obtained from the plurality of leads into samples having predetermined time length, removing noise of each of the samples, and evaluating quality of the each of the samples.

In an alternative embodiment, the removing of the noise of the each of the samples may include inputting the each of the samples into a bandpass filter, and removing, by the bandpass filter, at least one of high-frequency noise or lower-frequency noise.

In an alternative embodiment, the excluding of the electrocardiogram data obtained from the at least one lead among the plurality of leads may include determining that a quality of a sample of the at least one lead is lower than a threshold, and excluding the sample of the at least one lead determined to have the quality lower than the threshold in relation to analysis of a corresponding time segment.

In an alternative embodiment, the threshold may vary depending on at least one of a utilization purpose of the electrocardiogram data or an electrocardiogram measurement equipment.

In an alternative embodiment, the performing of the electrocardiogram analysis based on the remaining electrocardiogram data by using the pre-trained electrocardiogram analysis model may include performing the electrocardiogram analysis based on samples of remaining leads other than a sample of the at least one lead in relation to analysis of a corresponding time segment.

In an alternative embodiment, the performing of the electrocardiogram analysis based on the samples of the remaining leads other than the sample of the at least one lead in relation to the analysis of the corresponding time segment may include extracting feature values of the samples of the remaining leads through an encoder module, integrating the feature values in units of the corresponding time segment, and performing diagnosis by inputting the integrated feature value in a diagnosis determination module.

In an alternative embodiment, the encoder module may extract, when the electrocardiogram data is obtained from some leads of the plurality of leads, a feature value based on electrocardiogram data obtained from the some leads.

In an alternative embodiment, the encoder module may correspond to a module pre-trained based on labeling a sample in which there is a disease determined by the disease determination module, generating data for learning based on the labeled sample, and training the encoder module with the data for learning.

In an alternative embodiment, the disease determination module may include a classification sub module corresponding to each disease.

In an alternative embodiment, the performing of the diagnosis by inputting the integrated feature value in the diagnosis determination module may include deriving a probability value which is to correspond to each disease by inputting the integrated feature value in the diagnosis determination module, and diagnosing, by the disease determination module, whether there is the each disease according to whether the probability value is more than a threshold set for the each disease.

In order to implement the above-described object, according to an embodiment of the present disclosure, disclosed is a computer program for evaluating qualities of electrocardiogram data, and performing electrocardiogram analysis. The program may include: an operation of evaluating qualities of electrocardiogram data obtained from a plurality of leads; an operation of excluding electrocardiogram data obtained from at least one lead among the plurality of leads based on the evaluation of the qualities of the electrocardiogram data; and an operation of performing electrocardiogram analysis based on remaining electrocardiogram data by using a pre-trained electrocardiogram analysis model.

In order to implement the above-described object, according to an embodiment of the present disclosure, disclosed is a computing device for evaluating qualities of electrocardiogram data, and performing electrocardiogram analysis. The computing device may include: a processor including one or more cores; a network unit receiving one or more electrocardiogram data; and a memory, and the processor may evaluate qualities of electrocardiogram data obtained from a plurality of leads, exclude electrocardiogram data obtained from at least one lead among the plurality of leads based on the evaluation of the qualities of the electrocardiogram data, and perform electrocardiogram analysis based on remaining electrocardiogram data by using a pre-trained electrocardiogram analysis model.

### [Advantageous Effects]

The present disclosure can provide a disease diagnosis result by evaluating the quality of an electrocardiogram signal and using only a signal having excellent quality.

### [Description of Drawings]

The following accompanying drawings are only some of the embodiment of the present disclosure so as to be used for describing the embodiment of the present disclosure, and in the technical field of the present disclosure, those (hereinafter, referred to as "normal technician") skilled in the technical field of the present disclosure can obtain other drawings based on the drawings without an effort to reach a new invention.
FIG. 1 is a block diagram of a computing device for performing an operation of evaluating and analyzing electrocardiogram data according to an embodiment of the present disclosure.
FIG. 2 is a schematic diagram illustrating a network function according to an embodiment of the present disclosure.
FIG. 3 is a flowchart of a method for evaluating an electrocardiogram quality and diagnosing a disease through electrocardiogram analysis according to an embodiment of the present disclosure.
FIG. 4 is a conceptual diagram illustrating an electrocardiogram quality evaluating method according to an embodiment of the present disclosure.
FIG. 5 is a block diagram of a computing device according to an embodiment of the present disclosure.

### [Best Mode]

The present disclosure provides a method which evaluates the quality of electrocardiogram signals obtained from a plurality of leads, selects only data having excellent quality, and performs electrocardiogram analysis by using a deep learning model to stably diagnose a disease from the electrocardiogram signal under various situations.

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

In the present disclosure, a network function and an artificial neural network and a neural network may be interchangeably used.

Meanwhile, the terms "electrocardiogram data" used in the detailed description and claims of the present disclosure means data including a result of measuring electrocardiogram obtained from a plurality of leads constituted by multiple electrodes. The lead as induction may be defined as a trace in which a voltage difference between two different electrodes is recorded in electrocardiogram measurement equipment. For example, lead-1 is to record a voltage difference between an electrode of a right arm and an electrode of a left arm, and lead-2 is to record a voltage difference between a right arm and a right foot. In standard measurement equipment, 10 electrodes are used, so 12 leads may be formed, the number of leads and a data length of measured electrocardiogram data may vary depending on the electrocardiogram measurement equipment and a measurement time.

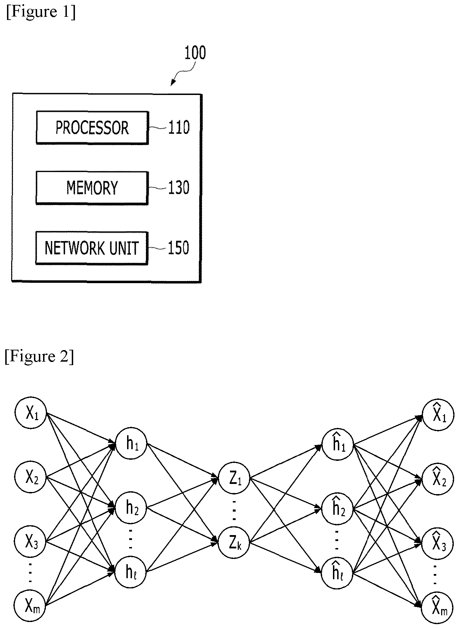

FIG. 1 is a block diagram of a computing device for performing an operation of evaluating and analyzing electrocardiogram data according to an embodiment of the present disclosure.

A configuration of the computing device 100 illustrated in FIG. 1 is only an example shown through simplification. In an embodiment of the present disclosure, the computing device 100 may include other components for performing a computing environment of the computing device 100, and only some of the disclosed components may also constitute the computing device 100.

The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an embodiment of the present disclosure. According to an embodiment of the present disclosure, the processor 110 may perform a calculation for learning the neural network. The processor 110 may perform calculations for learning the neural network, which include processing of input data for learning in deep learning (DL), extracting a feature in the input data, calculating an error, updating a weight of the neural network using backpropagation, and the like.

At least one of the CPU, GPGPU, and TPU of the processor 110 may process learning of a network function. For example, both the CPU and the GPGPU may process the learning of the network function and data classification using the network function. Further, in an embodiment of the present disclosure, processors of a plurality of computing devices may be used together to process the learning of the network function and the data classification using the network function. Further, the computer program executed in the computing device according to an embodiment of the present disclosure may be a CPU, GPGPU, or TPU executable program.

According to an embodiment of the present disclosure, the processor 110 may obtain one or more electrocardiogram data through a plurality of different types of paths. For example, the processor 110 may obtain electrocardiogram data received by an electrocardiogram device, obtain electrocardiogram data stored in the memory 130, or obtain the electrocardiogram data directly from equipment and the electrode for measuring the electrocardiogram, but the present disclosure is not limited thereto.

The processor 110 may perform a task of dividing the received electrocardiogram data into samples having predetermined time lengths. Since the electrocardiogram data used in the present disclosure is data measured for one hour as a short period and a long time corresponding to one week as a long period, the electrocardiogram data is divided into predetermined time units to analyze respective divided electrocardiogram data. The predetermined time length may be set independently of the number of leads and a measurement time length of equipment that measures the electrocardiogram, and may be a time length of a range to input the electrocardiogram data into an electrocardiogram analysis model for analyzing the electrocardiogram data.

The processor 110 may perform a task of removing noise of the electrocardiogram data divided into the samples having the predetermined time lengths. For example, a method for removing the noise may be performed by inputting the divided electrocardiogram data into a bandpass filter, and using an output value. The bandpass filter is a signal filter that passes only information in a desired specific frequency band without alleviation, and alleviates and transfers information outside the specific frequency band. In this case, high-frequency noise and low-frequency noise of the electrocardiogram data divided may be removed by the bandpass filter that alleviates a high frequency and a low frequency. However, the present disclosure is not limited to the noise removing method.

The processor 110 may evaluate the qualities of the respective electrocardiogram data divided into the predetermined time lengths, from which the noise is removed. For example, the quality evaluation of the electrocardiogram data may be performed by classifying the quality of the electrocardiogram data into a plurality of categories by a pre-trained neural network model, and scoring a specific class among respective classes, and also be performed by a rule based algorithm which does not use a model.

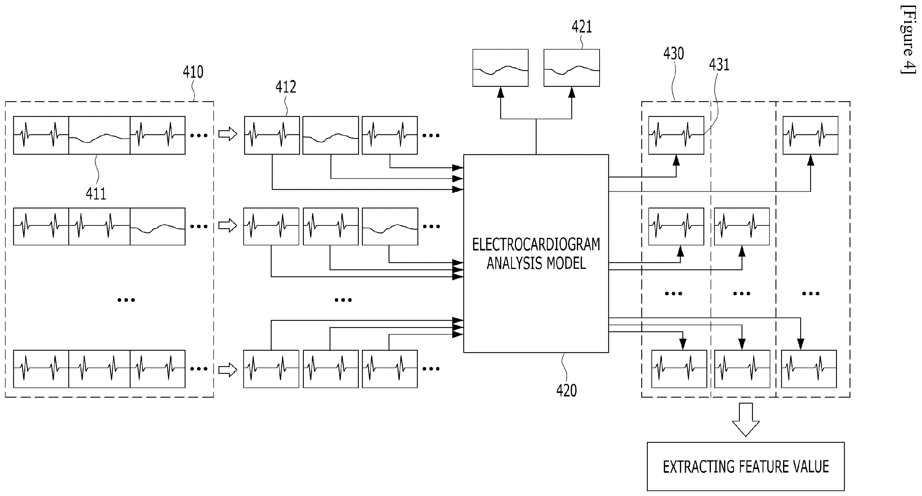

One of the methods that provides the method for scoring the electrocardiogram among electrocardiogram data related researches in the related art may be used or at least one of the methods presented in the related art may be combined and used, but the present disclosure is not limited thereto. A specific process of evaluating the qualities of the electrocardiogram data will be described below with reference to FIG. 4.

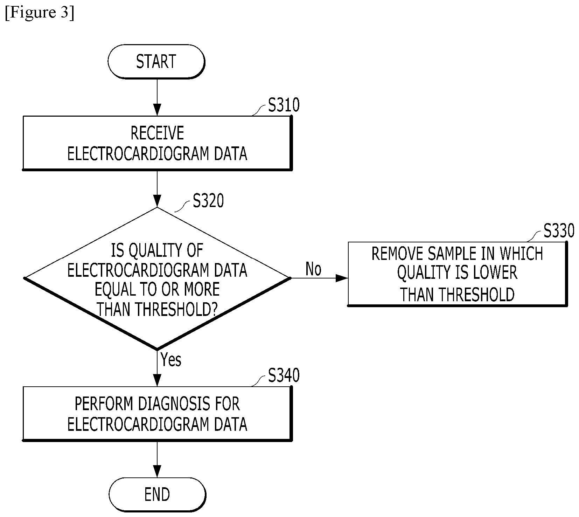

The processor 110 may exclude electrocardiogram data obtained from at least one lead among the electrocardiogram data obtained from the plurality of leads based on a result of evaluating the qualities of the electrocardiogram data. A specific method for excluding some electrocardiogram data will be described below with reference to FIG. 3.

The processor 110 may perform electrocardiogram analysis by utilizing a pre-trained electrocardiogram analysis model based on electrocardiogram data which remains after the excluding task. The electrocardiogram analysis model may include at least one encoder module and at least one disease determination module.

The processor 110 may extract a feature value corresponding to the number of respective samples even when the electrocardiogram data is obtained only in data from which samples of leads having low quality are removed from the electrocardiogram data divided by units of the time, i.e., some leads by using the encoder module included in the electrocardiogram analysis model.

For example, when the electrocardiogram data is obtained by using general 12-lead electrocardiogram measurement equipment, the electrocardiogram data is constituted by data of 12 leads having the same time length. The processor 110 may divide the electrocardiogram data at a predetermined time interval, and exclude a sample of at least one lead sample having low quality among the divided data. When it is assumed that two lead samples among 12 lead samples have the low quality and are excluded from the analysis, the encoder module may extract the feature value by analyzing the divided electrocardiogram data constituted by 10 leads.

In an embodiment of the present disclosure, when the electrocardiogram data is obtained only in some leads among the plurality of leads, the encoder module may extract the feature value by inferring single-lead encoders as large as the number of leads, and extract the feature value by inferring k-lead encoder of a number which is the same as the received number of leads once. When the example is continuously used, the encoder module is designed to include the single-lead encoder to extract 10 feature values by inferring the single-lead encoder 10 times in the electrocardiogram data of each lead, and designed to include a 10-lead encoder to extract 10 feature values through one inference. An n-lead encoder (n is an arbitrary natural number) mentioned in the present disclosure may be encoder module trained with electrocardiogram measurement data having n leads.

Further, the present disclosure includes a plurality of singe-lead encoders according to the type of lead to extract feature values to correspond to various types of leads. However, the feature value extraction method of the present disclosure is not limited to the above-described example. By such a method, according to the present disclosure, the feature values of the electrocardiogram data may be stably extracted from electrocardiogram data other than one or more electrocardiogram data having the low quality.

In an embodiment of the present disclosure, the single-lead encoder included in the encoder module may be pre-trained. The present disclosure discloses the method for diagnosing the disease by targeting the electrocardiogram having a long time length, but the learning method of the encoder module of the present disclosure is not limited, so it is also possible to use a machine learning module constructed for electrocardiogram analysis in the related art.

In an embodiment of the present disclosure, the processor 110 integrates the feature values extracted by the encoder module into one time segment to obtain one feature value. In a process of integrating the feature values, a neural network such as a fully-connected layer, a transformer, an attention-layer, etc., may also be used, and a mean of the respective feature values may be determined as the integrated feature value, but the present disclosure is not limited thereto.

In an embodiment of the present disclosure, the disease determination module included in the electrocardiogram analysis model may diagnose the disease by receiving the integrated feature value as an input. A specific process of diagnosing the disease will be described below with reference to FIG. 3.

The disease determination module may diagnose the disease and present the disease to the user, and for electrocardiogram data which is likely to correspond to the disease, a user who is an expert labels the disease to generate training data including labeled data from an output of the disease determination model. According to the present disclosure, the encoder module included in the electrocardiogram analysis model is additionally trained with the generated training data to increase the performance of the encoder model.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to several embodiments of the present disclosure may use various wired communication systems, such as a Public Switched Telephone Network (PSTN), an x Digital Subscriber Line (xDSL), a Rate Adaptive DSL (RADSL), a Multi Rate DSL (MDSL), a Very High Speed DSL (VDSL), a Universal Asymmetric DSL (UADSL), a High Bit Rate DSL (HDSL), and a local area network (LAN).

The network unit 150 presented in the present specification may use various wireless communication systems, such as Code Division Multi Access (CDMA), Time Division Multi Access (TDMA), Frequency Division Multi Access (FDMA), Orthogonal Frequency Division Multi Access (OFDMA), Single Carrier-FDMA (SC-FDMA), and other systems.

The network unit 150 according to an exemplary embodiment of the present disclosure may use an arbitrary type known wired/wireless communication systems.

The techniques described herein may be used in other networks in addition to those mentioned above.

FIG. 2 is a schematic diagram illustrating a network function according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be learned in at least one scheme of supervised learning, unsupervised learning, semi supervised learning, or reinforcement learning. The learning of the neural network may be a process in which the neural network applies knowledge for performing a specific operation to the neural network.

The neural network may be learned in a direction to minimize errors of an output. The learning of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data. That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a learning cycle (epoch). The learning rate may be applied differently according to the number of repetition times of the learning cycle of the neural network. For example, in an initial stage of the learning of the neural network, the neural network ensures a certain level of performance quickly by using a high learning rate, thereby increasing efficiency and uses a low learning rate in a latter stage of the learning, thereby increasing accuracy.

In learning of the neural network, the training data may be generally a subset of actual data (i.e., data to be processed using the learned neural network), and as a result, there may be a learning cycle in which errors for the training data decrease, but the errors for the actual data increase. Overfitting is a phenomenon in which the errors for the actual data increase due to excessive learning of the training data. For example, a phenomenon in which the neural network that learns a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the training data, regularization, dropout of omitting a part of the node of the network in the process of learning, utilization of a batch normalization layer, etc., may be applied.

FIG. 3 is a flowchart of a method for evaluating an electrocardiogram quality and diagnosing a disease through electrocardiogram analysis according to an embodiment of the present disclosure.

In step S310, the processor may receive electrocardiogram data on various paths including the electrocardiogram measurement equipment, the memory 130, or the network 150. Thereafter, a task of dividing the received electrocardiogram data into a predetermined time length, and removing noise may be performed.

In step S320, the processor 110 may evaluate the quality of the electrocardiogram data having the predetermined time length. For example, a quality score may be assigned to samples for each lead included in the electrocardiogram data having the predetermined time length, and respective samples may be classified based on a threshold.

In step S320, the threshold may be set differently according to a purpose. For example, the threshold may vary depending on the type of measurement equipment used for measuring the electrocardiogram data. As another example, even though the quality of the signal is sacrificed, there is a disease requiring a large amount of data, while even though the amount of data is small, there may be a disease in which an accurately measured signal is important for diagnosis, so the threshold in step S320 may be changed according to a disease to be determined. However, a reason for differently setting the threshold in the present disclosure is not limited thereto.

In step S330, when it is determined that the quality score of any sample is lower than the threshold, the corresponding sample is removed from the electrocardiogram data.

In step S340, when the quality score of any sample is equal to or more than the threshold in step S330, the corresponding sample corresponds to data which may be used for diagnosing the disease, so the corresponding sample is diagnosed.

The processor 110 may extract feature values of the remaining samples other than samples having the quality score which is lower than the threshold in order to analyze the electrocardiogram data by using the encoder module included in the electrocardiogram analysis model. After the features values of the samples are extracted by the encoder module, the processor 110 may integrate the feature values of the samples into one with respect to respective time intervals (i.e., respective time segments having the predetermined time length). For example, when there are nine samples in which the quality score is equal to or more than the threshold with respect to a first time interval and there are six samples in which the quality score is equal to or more than the threshold with respect to a second time interval, the feature value integrated for each time interval is the same as one regardless of the number of samples.

The processor 110 may determine the disease from the feature value for each time interval by using the disease determination module included in the electrocardiogram analysis model. For example, the disease determination module may include a classification sub module corresponding to each disease, and when the feature value is input, the disease determination module may derive a probability value in which each sub module is to correspond to the disease. Thereafter, the processor 110 may determine whether there is the disease according to whether the derived probability value is equal to or more than the threshold set for each disease. At this time, each classification sub module has a unique threshold, and the threshold may vary according to the disease to be diagnosed.

FIG. 4 is a conceptual diagram illustrating an electrocardiogram quality evaluating method according to an embodiment of the present disclosure.

The electrocardiogram data 410 includes lead-specific electrocardiogram data 411 as large as the number of leads. For example in the case of a basic -12-lead measurement machine, there are a total of lead-specific electrocardiogram data 411.

The processor 110- divides the electrocardiogram data 410 into a predetermined time length to obtain electrocardiogram data (not illustrated) of a time segment. The electrocardiogram data of each time segment includes samples 412 which are as many as leads having a predetermined time length.

The processor 110 may evaluate each electrocardiogram data (not illustrated) of a time segment by using an artificial neural network model or a rule based algorithm means 420. Thereafter, the processor 110 may exclude samples 421 in which the quality of the signal is lower than the threshold from the electrocardiogram data. At this time, the threshold may be changed according to the type of electrocardiogram measurement equipment and the disease to be diagnosed.

The processor 110 may obtain electrocardiogram data 430 constituted by the remaining samples 431 other than the samples 421 in which the quality of the signal is lower than the threshold by the above method, and then extract the feature value through the encoder module included in the electrocardiogram analysis model. At this time, the encoder module may extract the feature value by using only data other than electrocardiogram data having a low quality. For example, when two samples are excluded from the electrocardiogram data as a result of evaluating data of any time segment having 12 leads, the encoder module may extract 10 feature values from 10 remaining samples other than the corresponding samples. In the extraction process, as described above, various means including the method for inferring the single-lead encoder 10 times and the method for inferring tie 10-lead encoder once may be mobilized.

By such a method, the present disclosure excludes the data having the low quality from the electrocardiogram data to extract the feature value and use the feature value for determining the disease even though the electrocardiogram data exists in all leads.

In the meantime, according to an embodiment of the present disclosure, a computer readable medium storing a data structure is disclosed.

The data structure may refer to organization, management, and storage of data that enable efficient access and modification of data. The data structure may refer to organization of data for solving a specific problem (for example, data search, data storage, and data modification in the shortest time). The data structure may also be defined with a physical or logical relationship between the data elements designed to support a specific data processing function. A logical relationship between data elements may include a connection relationship between user defined data elements. A physical relationship between data elements may include an actual relationship between the data elements physically stored in a computer readable storage medium (for example, a permanent storage device). In particular, the data structure may include a set of data, a relationship between data, and a function or a command applicable to data. Through the effectively designed data structure, the computing device may perform a calculation while minimally using resources of the computing device. In particular, the computing device may improve efficiency of calculation, reading, insertion, deletion, comparison, exchange, and search through the effectively designed data structure.

The data structure may be divided into a linear data structure and a non-linear data structure according to the form of the data structure. The linear data structure may be the structure in which only one data is connected after one data. The linear data structure may include a list, a stack, a queue, and a deque. The list may mean a series of dataset in which order exists internally. The list may include a linked list. The linked list may have a data structure in which data is connected in a method in which each data has a pointer and is linked in a single line. In the linked list, the pointer may include information about the connection with the next or previous data. The linked list may be expressed as a single linked list, a double linked list, and a circular linked list according to the form. The stack may have a data listing structure with limited access to data. The stack may have a linear data structure that may process (for example, insert or delete) data only at one end of the data structure. The data stored in the stack may have a data structure (Last In First Out, LIFO) in which the later the data enters, the sooner the data comes out. The queue is a data listing structure with limited access to data, and may have a data structure (First In First Out, FIFO) in which the later the data is stored, the later the data comes out, unlike the stack. The deque may have a data structure that may process data at both ends of the data structure.

The non-linear data structure may be the structure in which the plurality of data is connected after one data. The non-linear data structure may include a graph data structure. The graph data structure may be defined with a vertex and an edge, and the edge may include a line connecting two different vertexes. The graph data structure may include a tree data structure. The tree data structure may be the data structure in which a path connecting two different vertexes among the plurality of vertexes included in the tree is one. That is, the tree data structure may be the data structure in which a loop is not formed in the graph data structure.

Throughout the present specification, a calculation model, a nerve network, the network function, and the neural network may be used with the same meaning. Hereinafter, the terms of the calculation model, the nerve network, the network function, and the neural network are unified and described with a neural network. The data structure may include a neural network. Further, the data structure including the neural network may be stored in a computer readable medium. The data structure including the neural network may also include preprocessed data for processing by the neural network, data input to the neural network, a weight of the neural network, a hyper-parameter of the neural network, data obtained from the neural network, an active function associated with each node or layer of the neural network, and a loss function for training of the neural network. The data structure including the neural network may include predetermined configuration elements among the disclosed configurations. That is, the data structure including the neural network may include the entirety or a predetermined combination of pre-processed data for processing by neural network, data input to the neural network, a weight of the neural network, a hyper parameter of the neural network, data obtained from the neural network, an active function associated with each node or layer of the neural network, and a loss function for training the neural network. In addition to the foregoing configurations, the data structure including the neural network may include predetermined other information determining a characteristic of the neural network. Further, the data structure may include all type of data used or generated in a computation process of the neural network, and is not limited to the foregoing matter. The computer readable medium may include a computer readable recording medium and/or a computer readable transmission medium. The neural network may be formed of a set of interconnected calculation units which are generally referred to as "nodes". The "nodes" may also be called "neurons." The neural network consists of one or more nodes.

The data structure may include data input to the neural network. The data structure including the data input to the neural network may be stored in the computer readable medium. The data input to the neural network may include training data input in the training process of the neural network and/or input data input to the training completed neural network. The data input to the neural network may include data that has undergone pre-processing and/or data to be pre-processed. The pre-processing may include a data processing process for inputting data to the neural network. Accordingly, the data structure may include data to be pre-processed and data generated by the pre-processing. The foregoing data structure is merely an example, and the present disclosure is not limited thereto.

The data structure may include a weight of the neural network (in the present specification, weights and parameters may be used with the same meaning), Further, the data structure including the weight of the neural network may be stored in the computer readable medium. The neural network may include a plurality of weights. The weight is variable, and in order for the neural network to perform a desired function, the weight may be varied by a user or an algorithm. For example, when one or more input nodes are connected to one output node by links, respectively, the output node may determine a data value output from the output node based on values input to the input nodes connected to the output node and the weight set in the link corresponding to each of the input nodes. The foregoing data structure is merely an example, and the present disclosure is not limited thereto.

For a non-limited example, the weight may include a weight varied in the neural network training process and/or the weight when the training of the neural network is completed. The weight varied in the neural network training process may include a weight at a time at which a training cycle starts and/or a weight varied during a training cycle. The weight when the training of the neural network is completed may include a weight of the neural network completing the training cycle. Accordingly, the data structure including the weight of the neural network may include the data structure including the weight varied in the neural network training process and/or the weight when the training of the neural network is completed. Accordingly, it is assumed that the weight and/or a combination of the respective weights are included in the data structure including the weight of the neural network. The foregoing data structure is merely an example, and the present disclosure is not limited thereto.

The data structure including the weight of the neural network may be stored in the computer readable storage medium (for example, a memory and a hard disk) after undergoing a serialization process. The serialization may be the process of storing the data structure in the same or different computing devices and converting the data structure into a form that may be reconstructed and used later. The computing device may serialize the data structure and transceive the data through a network. The serialized data structure including the weight of the neural network may be reconstructed in the same or different computing devices through deserialization. The data structure including the weight of the neural network is not limited to the serialization. Further, the data structure including the weight of the neural network may include a data structure (for example, in the non-linear data structure, B-Tree, Trie, m-way search tree, AVL tree, and Red-Black Tree) for improving efficiency of the calculation while minimally using the resources of the computing device. The foregoing matter is merely an example, and the present disclosure is not limited thereto.

The data structure may include a hyper-parameter of the neural network. The data structure including the hyper-parameter of the neural network may be stored in the computer readable medium. The hyper-parameter may be a variable varied by a user. The hyper-parameter may include, for example, a learning rate, a cost function, the number of times of repetition of the training cycle, weight initialization (for example, setting of a range of a weight value to be weight-initialized), and the number of hidden units (for example, the number of hidden layers and the number of nodes of the hidden layer). The foregoing data structure is merely an example, and the present disclosure is not limited thereto.

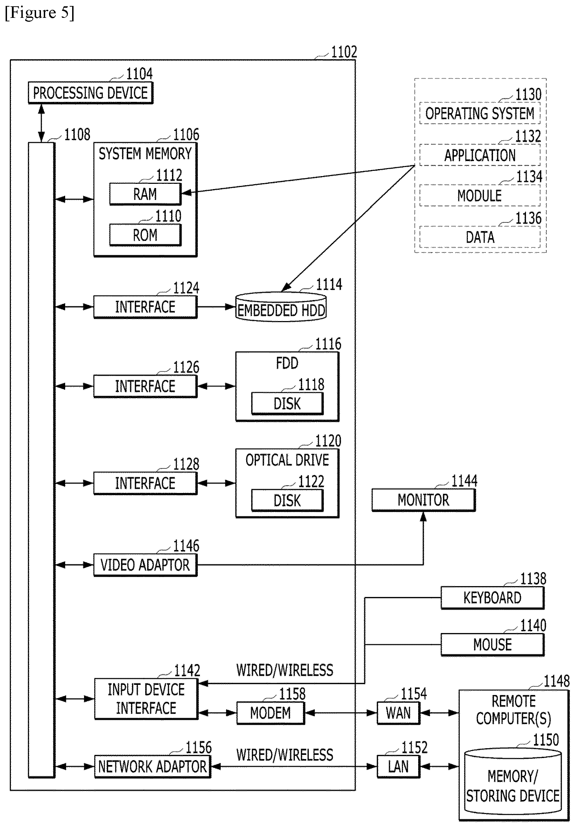

FIG. 5 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

In general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multiprocessor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.1 la) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

## Claims

1. A method performed by one or more processors of a computing device, the method comprising:
evaluating qualities of electrocardiogram data obtained from a plurality of leads;
excluding electrocardiogram data obtained from at least one lead among the plurality of leads based on the evaluation of the qualities of the electrocardiogram data; and
performing electrocardiogram analysis based on remaining electrocardiogram data by using a pre-trained electrocardiogram analysis model.

2. The method of claim 1, wherein the evaluating of the qualities of the electrocardiogram data obtained from the plurality of leads includes:
dividing the electrocardiogram data obtained from the plurality of leads into samples having predetermined time length,
removing noise of each of the samples, and
evaluating quality of the each of the samples.

3. The method of claim 2, wherein the removing of the noise of the each of the samples includes:
inputting the each of the samples into a bandpass filter, and
removing, by the bandpass filter, at least one of high-frequency noise or lower-frequency noise.

4. The method of claim 2, wherein the excluding of the electrocardiogram data obtained from the at least one lead among the plurality of leads includes:
determining that a quality of a sample of the at least one lead is lower than a threshold, and
excluding the sample of the at least one lead determined to have the quality lower than the threshold in relation to analysis of a corresponding time segment.

5. The method of claim 4, wherein the threshold varies depending on at least one of a utilization purpose of the electrocardiogram data or an electrocardiogram measurement equipment.

6. The method of claim 1, wherein the performing of the electrocardiogram analysis based on the remaining electrocardiogram data by using the pre-trained electrocardiogram analysis model includes:
performing the electrocardiogram analysis based on samples of remaining leads other than a sample of the at least one lead in relation to analysis of a corresponding time segment.

7. The method of claim 6, wherein the performing of the electrocardiogram analysis based on the samples of the remaining leads other than the sample of the at least one lead in relation to the analysis of the corresponding time segment includes:
extracting feature values of the samples of the remaining leads through an encoder module,
integrating the feature values in units of the corresponding time segment, and
performing diagnosis by inputting the integrated feature value in a diagnosis determination module.

8. The method of claim 7, wherein the encoder module extracts, when electrocardiogram data is obtained from some leads of the plurality of leads, a feature value based on the electrocardiogram data obtained from the some leads.

9. The method of claim 7, wherein the encoder module corresponds to a module pre-trained based on:
labeling a sample in which there is a disease determined by the disease determination module,
generating data for learning based on the labeled sample, and
training the encoder module with the data for learning.

10. The method of claim 7, wherein the disease determination module includes a classification sub module corresponding to each disease.

11. The method of claim 7, wherein the performing of the diagnosis by inputting the integrated feature value in the diagnosis determination module includes:
deriving a probability value which is to correspond to each disease by inputting the integrated feature value in the diagnosis determination module, and
diagnosing, by the disease determination module, whether there is the each disease according to whether the probability value is more than a threshold set for the each disease.

12. A computer program stored in a computer readable storage medium, the computer program including instructions which cause a computing device to perform operations, the operations comprising:
an operation of evaluating qualities of electrocardiogram data obtained from a plurality of leads;
an operation of excluding electrocardiogram data obtained from at least one lead among the plurality of leads based on the evaluation of the qualities of the electrocardiogram data; and
an operation of performing electrocardiogram analysis based on remaining electrocardiogram data by using a pre-trained electrocardiogram analysis model.

13. A computing device comprising:
a processor including one or more cores;
a network unit receiving one or more electrocardiogram data; and
a memory,
wherein the processor is configured to:
evaluates qualities of electrocardiogram data obtained from a plurality of leads,
excludes electrocardiogram data obtained from at least one lead among the plurality of leads based on the evaluation of the qualities of the electrocardiogram data, and
performs electrocardiogram analysis based on remaining electrocardiogram data by using a pre-trained electrocardiogram analysis model.
